Europäisches Patentamt

European Patent Office

Office européen des brevets

(11) Publication number: 0 278 513 B1

## EUROPEAN PATENT SPECIFICATION

(45) Date of publication of patent specification: 30.09.92    (51) Int. Cl.⁵: **C12N 15/11, C12P 19/34**

(21) Application number: **88102033.3**

(22) Date of filing: **11.02.88**

(54) Process of preparing single-stranded DNA.

(30) Priority: **12.02.87 JP 30420/87**

(43) Date of publication of application:
**17.08.88 Bulletin 88/33**

(45) Publication of the grant of the patent:
**30.09.92 Bulletin 92/40**

(84) Designated Contracting States:
**DE FR GB IT NL**

(56) References cited:
**EP-A- 0 201 184**
**WO-A-87/04165**

**CHEMICAL ABSTRACTS, vol. 97, no. 5, 2nd August 1982, page 131, abstract no. 34130k, Columbus, Ohio, US; N.T. HU et al.: "The making of strand-specific M13 probes", & GENE 1982, 17(3), 271-7**

(73) Proprietor: **TORAY INDUSTRIES, INC.**
**2-1, Nihonbashi Muromachi 2-chome Chuo-ku**
**Tokyo 103(JP)**

(72) Inventor: **Murao, Yasuo**
**K-202, 2-1-17, Tsunishi**
**Kamakura-shi Kanagawa 248(JP)**
Inventor: **Hosaka, Shuntaro**
**3-11-2, Jindaiji**
**Mitaka-sji Tokyo 181(JP)**

(74) Representative: **Kador & Partner**
**Corneliusstrasse 15**
**W-8000 München 5(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

# Description

## BACKGROUND OF THE INVENTION

### I. Field of the Invention

This invention relates to a process of preparing single-stranded DNAs. The single-stranded DNAs may suitably be used for DNA probes for detecting a DNA in interest.

### II. Description of the Prior Art

The base sequence of a DNA or RNA is unique to the virus or the organism containing the DNA or RNA. The DNA or RNA specifically hybridizes with a DNA or RNA which is complementary thereto to form a double-stranded chain (hybrid). Recently, utilizing this property, DNA probes for detecting or quantifying DNAs and RNAs were developed and the testing kits utilizing the DNA probes are commercially available.

In detecting a DNA or RNA using such a probe, a porous nitrocellulose membrane or porous Nylon membrane is typically used as an adsorbent for adsorbing DNA or RNA. That is, firstly, a test sample solution is spotted on, or passing through the adsorbent membrane to adsorb the DNA or RNA to be detected on the membrane, followed by heating the membrane to fix the DNA or RNA to be detected. Then the adsorbent membrane is treated with a DNA or RNA such as salmon sperm DNA which is not complementary to the DNA or RNA to be detected so as to ensure that the probe is not adsorbed on the membrane non-specifically. Then the membrane is treated with a DNA probe which contains a base sequence complementary to at least a portion of the DNA or RNA to be detected, and which has a detectable marker or a functional group which may be labelled with a marker. If the DNA or RNA to be detected exists on the membrane, the DNA probe hybridizes therewith to form a hybrid. After treatment with the probe, the membrane is washed to remove the non-hybridized DNA probe. The probes which formed hybrids are then detected utilizing the marker which is carried on the probe or which may be introduced in the probe to detect the DNA or RNA to be detected.

Most of the conventional DNA probes are constructed by recombining a DNA from a virus, microorganism or animal or plant cell, which is to be detected, in an appropriate vector such as pBR322 plasmid vector and M13RF phage vector. Thus, most of the conventional DNA probes contain a DNA region originated from the vector, and are double-stranded. In cases where the DNA probe contains a DNA region originated from the vector, if the test sample is contaminated by a DNA from the same origin as the vector, the probe is hybridized with the contaminant DNA to present a false-positive result. Further, since the larger the DNA molecule, the more likely the DNA adsorbed on the adsorbent membrane, the conventional DNA probes which are large due to the existence of the DNA region originated from the vector are likely to be non-specifically adsorbed on the membrane to present high background. Further, because of the large size of the probe, it takes a long time to make the probe hybridize with the DNA or RNA to be detected, so that a long time is needed for the test accordingly. Still further, if the probe is double-stranded, the efficiency of the hybridization with the DNA or RNA to be detected is lowered. Therefore, the probe is preferably single-stranded.

## SUMMARY OF THE INVENTION

Accordingly, the object of the present invention is to provide a process of conveniently and efficiently preparing a single-stranded DNA which does not contain undesired region such as vector-derived region, which single-stranded DNA, when used as a DNA probe, can reduce the non-specific adsorption and which can shorten the time required for the detection of the DNA or RNA to be detected.

In the first step of the present invention, a single-stranded DNA containing a template DNA region is provided. The template DNA has a base sequence complementary to that of the DNA to be prepared. In cases where the single-stranded DNA is used as a DNA probe, the template DNA region has a base sequence which is to be detected by the probe. Thereafter, means for terminating extension of DNA chain which is to be formed using the template DNA as a template in a later step is provided at the 5′ end portion of the template DNA. Then at least one primer is hybridized with a portion of the template DNA. Thereafter, the primer is extended utilizing the template DNA as a template. Finally, the single-stranded DNA formed by the extension of the primer is separated and recovered.

By the method of the present invention, single-stranded DNAs may be prepared conveniently and efficiently. If the single-stranded DNA prepared by the process of the present invention is used as a DNA probe, since the single-stranded DNA does not contain vector-derived region, the false-positive result due to the contamination by a DNA of the same origin as the vector may be avoided. Further, since the size of the single-stranded DNA is small, non-specific adsorption to the adsorbent membrane is reduced. Thus, the single-stranded DNA prepared by the method of the present invention can be used as a sensitive DNA probe.

## BRIEF DESCRIPTION OF THE DRAWING

The figure schematically shows one embodiment of the process of the present invention.

## DETAILED DESCRIPTION OF THE PREFERRED EMBODIMENTS

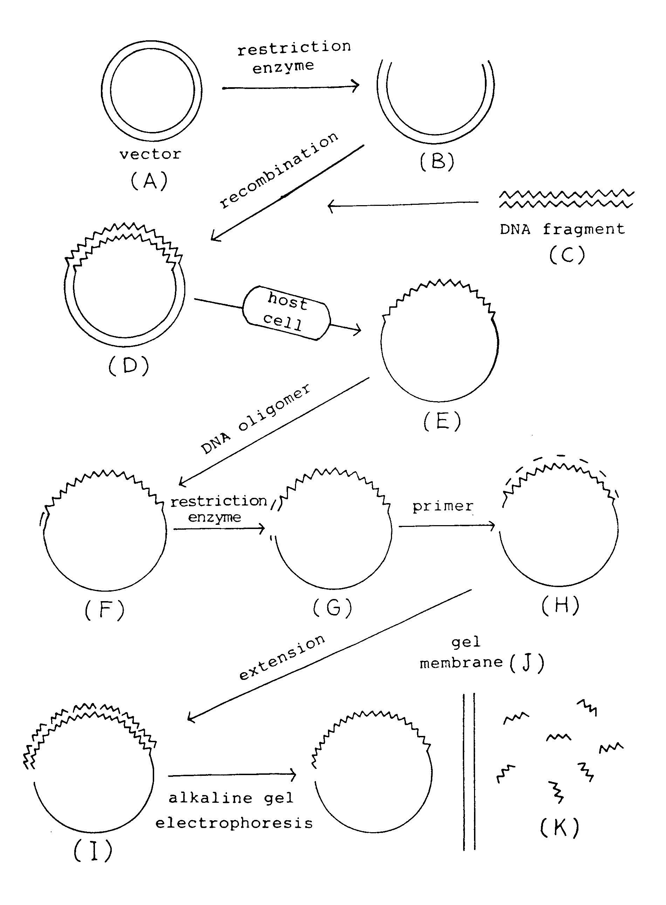

As mentioned above, in the first step of the process of the present invention, a single-stranded DNA containing a template DNA region having a base sequence complementary to that of the single-stranded DNA to be prepared is provided. In cases where the prepared single-stranded DNA is to be used as a DNA probe, the template DNA region has a base sequence which is substantially the same as that of a portion of the DNA or RNA to be detected. The single-stranded DNA containing the template DNA may be prepared, for example, as follows: Firstly, as shown in Fig. 1, a double-stranded vector (A) is digested with an appropriate restriction enzyme to cleave the vector (B). As is well-known, some phage vectors such as M13 series and $\phi$X174 release single-stranded circular DNA out of a host cell in which they are in the replication form (RF), so that such a vector may preferably be used. On the other hand, a double-stranded DNA fragment (C) is prepared by cutting a DNA with the same restriction enzyme used for cleaving the vector. One of the chains of the DNA fragment (C) is the DNA to be prepared by the process of the present invention. Thus, when the single-stranded DNA prepared by the process of the present invention is used as a DNA probe, the fragment (C) is derived from a virus, microorganism, animal cell, plant cell or the like, which is desired to be detected. Then the cleaved vector (B) and the DNA fragment (C) are recombined to form a double-stranded recombinant vector (D). The recombination procedure per se is well-known in the art. The recombinant vector is then transfected in a host cell such as E. coli cell. The transfection procedure is also well-known in the art. By culturing the host cell, single-stranded recombinant DNA (E) is released from the host cell.

In the second step, means for terminating the extension of DNA chain which is to be formed using the template DNA as a template in a later step is provided at the 5′ end portion of the template DNA. In a preferred embodiment, the means for terminating the extension of the DNA chain is provided by cutting the 5′ end portion of the template DNA. The extension of the DNA chain is terminated at the cut site. Cutting the 5′ end portion of the template DNA may be conducted by first hybridizing a DNA oligomer with the 5′ end portion of the template DNA to form a double-stranded portion (Fig. 1 (F)) and then cutting the thus formed double-stranded portion with a restriction enzyme having a restriction site in the double-stranded portion (Fig. 1 (G)). The DNA oligomer preferably has 10 to 20 bases and most preferably 15 bases.

Then one or more primers are hybridized with a portion of the template DNA (Fig. 1 (H)). The primer preferably contain 10 to 20 bases, and more preferably 15 to 17 bases. In a preferred embodiment, a plurality of primers are hybridized. This is preferred because a plurality of single-stranded DNA may be prepared in a single process. In cases where a plurality of the primers are hybridized, it is preferred that the primers are hybridized at the portion with an interval of 100 to 1,000 bases, more preferably 150 to 500 bases. It is also preferred that the primers are hybridized at the portions with substantially uniform intervals. Since the primer is complementary to the portion of the template DNA with which it is hybridized, the base sequence must be known at least for the portion.

Then the one or more primers are extended using large fragment of the DNA polymerase I in the presence of four kinds of deoxyribonucleoside triphosphate, i.e., dATP, dTTP, dGTP and dCTP (Fig. 1 (I)). This extension process is well-known in the art. The DNA chains formed by the extension of the primers are the desired DNAs.

Finally, the DNAs thus formed by the extension of the primers are then separated from the template DNA and recovered. In a preferred embodiment, separation and recovery of the formed single-stranded DNAs is conducted by alkaline gel electrophoresis. That is, as shown in Fig. 1 (J), a chamber is separated by a gel membrane such as agarose gel and acrylamide gel membrane. In one chamber, an alkaline solution containing the partially double-stranded DNA formed in the preceding step is placed. The thickness of the gel membrane is preferably 1 - 10 mm, and more preferably 2 - 5 mm. A basic substance such as NaOH is added to the solution to denature the double-stranded portion. Then a voltage is applied across the gel membrane such that the negative voltage is applied to the DNA solution to conduct electrophoresis. The voltage may be, for example, 50V to 100V. The duration of the electrophoresis depends on the voltage applied and, for example, 20 minutes when the voltage is 70V. By the electrophoresis, as shown in Fig. 1 (K), the desired single-stranded DNAs migrate through the gel membrane into another chamber, while the single-stranded DNA containing the template DNA remains in the chamber in which the DNA solution is originally placed.

In cases where the single-stranded DNA formed by the process of the present invention is used as a DNA probe, the single-stranded DNA must be able to be detected. The prepared single-stranded DNA may be labelled by a well-known conventional method. That is, the prepared single-stranded DNA may be labelled with a radioactive marker, enzyme, biotin and the like. For example, the prepared single-stranded DNA may be treated with Photobiotin (commercially available from Biotechnology Research Enterprizes) or Chemiprobe (commercially available from Orgenics, Ltd.). Alternatively, in a more preferred embodiment, a deoxyribonucleoside triphosphate with a marker such as radio active marker, enzyme, biotin and a hapten, or a functional group such as amino group or carboxyl group to which a marker such as a hapten, biotin and enzyme can be attached later is used for the extension of the primer. When a deoxyribonucleoside triphosphate labelled with a marker is used for the extnesion of the primer, the prepared single-stranded DNA is labelled with the marker, accordingly. If a deoxyribonucleoside triphosphate with a functional group to which a marker can be attached is used, the prepared single-stranded DNA may easily be labelled with a marker. In view of the fact that the extension of the primer is not hindered by the presence of the marker, it is preferred that the doxyribonucleoside triphosphate have a functional group to which a marker can be attached later rather than it has a marker. The labelling of the DNA and the introduction of the functional group into a deoxyribonucleoside triphosphate per se are known in the art and are extensively discussed in, for example, an International Application Published under the PCT numbered WO87/04165 which is hereby incorporated by reference. The labelled single-stranded DNA can be used as a DNA probe in the conventional manner.

The present invention will now be described by way of examples thereof. These examples are presented for the illustration purpose only, and should not be interpreted any restictive way.

Example 1

(1) Preparation of M13mp19 single-stranded DNA in which hepatitis B virus (HBV) is inserted (HB/M13)

In accordance with the method described in "Cloning and Dideoxy Sequence Method Using M13 Phage", pp.24 - 31, Published from Amersham Japan, January 1, 1984. HB/M13 in which 1.4 kb DNA fragment cut out from HBV with a restriction enzyme BamHI was obtained.

(2) Cleavage and ring-opening of the HB/M13

Fifteen microliters of a solution of DNA oligomer with a base sequence of

5'AAGCTTGCATGCCTG3' with a concentration of 5 O.D./ml at 260 nm and 100 $\mu$l of HB/M13 with a concentration of 5 $\mu$g/$\mu$l were mixed and the mixture was incubated for 5 minutes at 55°C and then for 5 minutes at room temperature to hybridize the DNA oligomer with the 5' cloning site. The above-described base sequence of the oligomer contains a restriction site of HindIII. Thereafter, $MgCl_2$ and NaCl were added to the mixture to a final concentration of 7 mM and 60 mM, respectively. Then 8 $\mu$l (10 units/$\mu$l) of HindIII was added to the mixture and the mixture was incubated at 37°C for 3 hours to cut the double-stranded portion of the HB/M13 hybridized with the DNA oligomer.

(3) Hybridization of primers and extension thereof

Fifteen microliters of six solutions each containing a DNA oligomer (primer) which has a base sequence of 5'GCGGCTAGGAGTTCC3', 5'CCTGTTTAGCTTGTA3', 5'ATGTAGCCCATGAAG3', 5'AATGGCACTAGTAAA3', 5'TGGAATTAGAGGACA3' or 5'TTGAGAGAAGTCCAC3' were mixed with the solution containing the ring-opened HB/M13 obtained in (2) and the mixture was incubated at 55°C for 5 minutes and at room temperature for 5 minutes, thereby hybridizing the primer with the portions of the HBV-derived region of the HB/M13, which portions being located at 200 bases intervals from the 5' end of the HBV-derived region. Thereafter, 100 $\mu$l of a buffer solution (67 mM potassium phosphate, 6.7 mM $MgCl_2$, pH7.4) containing 1 mM each of dATP, dCTP, dGTP and dTTP, and 3 $\mu$l of DNA polymerase large fragment (4.2 units/$\mu$l) were added and the mixture was incubated at 25°C for 5 minutes, thereby extending the primers. Then 250 mM aqueous solution of EDTA was added and the DNA was recovered by phenol extraction and ethanol percipitation.

(4) Separation and recovery of the extended DNA

A chamber made of a cellulose semipermeable membrane was separated by a 3% agarose gel membrane to form two chambers. The DNA solution obtained in (3) containing 50 mM NaOH and 1 mM EDTA was placed in one chamber, and an elecrophoresis solution containing 30 mM NaOH and 1 mM EDTA was placed in another chamber. Then 70V of electric voltage was applied across the gel membrane for 20 minutes such that the negative voltage was applied to the chamber in which the DNA solution is contained. By this electrophoresis, single-stranded DNAs each containing 200 bases were obtained. This was confirmed by the typical conventional gel electrophoresis using a marker.

Example 2

The same procedure as in Example 1 was repeated except that 5 μl of 1 mM aqueous solution of allylamine-dUTP (prepared in accordance with the teaching in Proc. Natl. Acad. Sci. USA Vol. 78, No. 11, pp.6633 - 6637, November 1981) was added to the mixture used in extending the primer.

After the single-stranded DNAs were recovered as in Example 1, the solution containing the single-stranded DNAs was dialyzed against 0.1 M $NaHCO_3$ solution to obtain a 0.1 M $NaHCO_3$ solution containing the single-stranded DNAs. To 900 μl of this solution, 25 ul of a solution of ε-caproylamidobiotin-N-hydroxysuccinimide ester (commercially available from BRL, USA) (10 μg/μl) in DMF was added and the mixture was stirred at room temperature for 1 hour. Thereafter, the mixture was dialyzed against TE buffer (10 mM Tris-HCl, 1 mM EDTA, pH = 8.0). The resulting solution was spotted on a nitrocellulose membrane and the spots were assayed with acid phosphatase avidin conjugate to confirm that biotin was bonded to the single-stranded DNA.

**Claims**

1. A process of preparing a single-stranded DNA comprising the steps of:
   providing a single-stranded DNA containing a template DNA region having a base sequence complementary to that of the single-stranded DNA to be prepared;
   providing, at the 5′ end portion of the template DNA, means for terminating extension of DNA chain which is to be formed using the template DNA as a template in a later step;
   hybridizing at least one primer with a portion of the template DNA;
   extending the primer utilizing the template DNA as a template; and
   separating and recovering single-stranded DNA formed by the extension of the primer.

2. The process of claim 1, wherein the means for terminating the extension of the DNA chain is provided by cutting the 5′ end portion of the template DNA.

3. The process of claim 2, wherein the 5′ end portion of the template DNA is cut by hybridizing a DNA oligomer with the 5′ end portion of the template DNA to form a double-stranded portion and then cutting the thus formed double-stranded portion with a restriction enzyme having a restriction site in the double-stranded portion.

4. The process of claim 1, wherein a plurality of the primers are hybridized with the template DNA.

5. The process of claim 4, wherein the primers are hybridized with portions of the template DNA with an interval of 100 to 1000 bases.

6. The process of claim 5, wherein the interval between the primers are 150 to 500 bases.

7. The process of claim 4, wherein the primers are hybridized with portions of the template DNA with substantially uniform intervals.

8. The process of claim 1, further comprising the step of labelling the formed single-stranded DNA.

9. The process of claim 1, wherein the extension of the primer is conducted utilizing deoxyribonucleoside triphosphate with a marker or a functional group to which a marker can be attached.

10. The process of claim 9, wherein the extension of the primer is conducted utilizing deoxyribonucleoside triphosphate with a functional group to which a marker can be attached.

11. The process of claim 10, wherein the functional group is amino group or carboxyl group.

12. The process of claim 10, wherein the marker is biotin.

13. The process of claim 1, wherein the separation and recovery of the single-stranded DNA is conducted by alkaline gel electrophoresis utilizing a gel membrane.

14. A process of preparing a single-stranded DNA comprising the steps of:
   recombining a phage vector which releases single-stranded form phage with a template DNA having a base sequence complementary to that of the single-stranded DNA to be prepared;
   transfecting a host cell by the recombinant DNA;
   culturing the host cell to release a single-stranded DNA which is a recombinant of the phage vector and the template DNA;
   hybridizing a DNA oligomer with the 5′ end portion of the template DNA to form a double-stranded region at the 5′ end portion of the template DNA;
   cutting the thus formed double-stranded region with a restriction enzyme to cut the 5′ end portion of the template DNA;

hybridizing a plurality of primers with portions of the template DNA which are substantially uniformly spaced,

extending the primers utilizing the template DNA as a template; and

separating and recovering single-stranded DNAs formed by the extension of the primers.

## Patentansprüche

1. Verfahren zur Herstellung von einzelsträngiger DNS, welches die Schritte umfaßt:

Bereitstellung einer einzelsträngigen DNS, die einen DNS-Templatbereich mit einer Basenfolge enthält, die der der herzustellenden einzelsträngigen DNS komplementär ist;

Schaffung einer Maßnahme am 5'-Endabschnitt des DNS-Templates zum Abschluß des Wachstums der zu bildenden DNS-Kette, wobei das DNS-Templat in einem späteren Schritt als Templat verwendet wird;

Hybridisierung von zumindest einem Primer mit einem Teil des DNS-Templates;

Verlängerung des Primers, wobei das DNS-Templat als Templat verwendet wird; und

Abtrennung und Gewinnung der durch die Verlängerung des Primers gebildeten einzelsträngigen DNS.

2. Verfahren nach Anspruch 1, worin die Maßnahme zum Abschluß des Wachstums der DNS-Kette durch Abtrennen des 5'-Endabschnittes des DNS-Templates geschaffen wird.

3. Verfahren nach Anspruch 2, worin der 5'-Endabschnitt des DNS-Templates durch Hybridisierung eines DNS-Oligomers mit dem 5'-Endabschnitt des DNS-Templates abgetrennt wird, um einen doppelsträngigen Abschnitt zu bilden, und danach der so gebildete doppelsträngige Abschnitt mit einem Restriktionsenzym abgetrennt wird, das im doppelsträngigen Abschnitt eine Restriktionsstelle hat.

4. Verfahren nach Anspruch 1, worin eine Vielzahl von Primeren mit dem DNS-Templat hybridisiert wird.

5. Verfahren nach Anspruch 1, worin die Primer mit Abschnitten des DNS-Templates mit einem Intervall von 100 bis 1000 Basen hybridisiert werden.

6. Verfahren nach Anspruch 5, worin der Intervall zwischen den Primer 150 bis 500 Basen beträgt.

7. Verfahren nach Anspruch 4, worin die Primer mit Abschnitten des DNS-Templates mit im wesentlichen einheitlichen Intervallen hybridisiert werden.

8. Verfahren nach Anspruch 1, das außerdem den Schritt der Kennzeichnung der gebildeten einzelsträngigen DNS umfaßt.

9. Verfahren nach Anspruch 1, worin die Verlängerung des Primers unter Verwendung von Desoxyribonukleosidtriphosphat mit einer Markierung oder einer funktionellen Gruppe, an der die Markierung angefügt werden kann, durchgeführt wird.

10. Verfahren nach Anspruch 9, worin die Verlängerung des Primers unter Anwendung von Desoxyribonukleosidtriphosphat mit einer funktionellen Gruppe durchgeführt wird, an die die Markierung angefügt werden kann.

11. Verfahren nach Anspruch 10, worin die funktionelle Gruppe eine Aminogruppe oder eine Carboxylgruppe ist.

12. Verfahren nach Anspruch 10, worin die Markierung Biotin ist.

13. Verfahren nach Anspruch 1, worin die Abtrennung und Gewinnung der einzelsträngigen DNS durch alkalische Gel-Elektrophorese durchgeführt wird, wobei eine Gelmembran angewendet wird.

14. Verfahren zur Herstellung einer einzelsträngigen DNS, welches die Schritte umfaßt:

Wiedervereinigung eines Phagenvektors, der eine Phage in Einzelstrangform freisetzt, mit einem DNS-Templat, das eine Basenfolge aufweist, die der der herzustellenden einzelsträngigen DNS komplementär ist;

Transfektion einer Wirtszelle durch die rekombinierte DNS;

Kultivierung der Wirtszelle, um die einzelsträngige DNS freizusetzen, die eine Wiedervereinigung des Phagenvektors und des DNS-Templates darstellt;

Hybridisierung des DNS-Oligomers mit dem 5'-Endabschnitt des DNS-Templates, um einen doppelsträngigen Bereich am 5'-Endabschnitt des DNS-Templates zu bilden;

Abtrennen des so gebildeten doppelsträngigen Bereiches mit einem Restriktionsenzym, um den 5'-Endabschnitt des DNS-Templates abzutrennen;

Hybridisierung einer Vielzahl von Primer mit Abschnitten des DNS-Templates, die einen im wesentlichen gleichmäßigen Abstand aufweisen;
Verlängerung der Primer, wobei das DNS-Templat als Templat verwendet wird, und
Abtrennung und Gewinnung der durch Verlängerung der Primer gebildeten einzelsträngigen DNS.

## Revendications

1. Procédé de préparation d'un ADN à simple brin comprenant les stades consistant à :
   - réaliser un ADN à simple brin contenant une région d'ADN matrice ayant une séquence complémentaire de celle de l'ADN à simple brin à préparer;
   - réaliser, dans la portion d'extrémité 5' de l'ADN de matrice, un moyen pour mettre fin à l'allongement de la chaîne d'ADN qui doit être formée en utilisant l'ADN matrice comme matrice dans un stade ultérieur;
   - hybrider au moins un primaire avec une portion de l'ADN matrice;
   - allonger le primaire en utilisant l'ADN matrice comme matrice; et
   - séparer et récupérer l'ADN à simple brin formé par l'allongement du primaire.

2. Procédé selon la revendication 1, dans lequel le moyen pour terminer l'allongement de la chaîne d'ADN est réalisé par coupure de la portion d'extrémité 5' de l'ADN matrice.

3. Procédé selon la revendication 2, dans lequel la portion d'extrémité 5' de l'ADN matrice est coupée en hybridant un oligomère d'ADN avec la portion d'extrémité 5' de l'ADN matrice pour former une portion à double brin, puis en coupant la portion à double brin ainsi formée avec une enzyme de restriction ayant un site de restriction dans la portion à double brin.

4. Procédé selon la revendication 1, dans lequel on hybride plusieurs primaires avec l'ADN matrice.

5. Procédé selon la revendication 4, dans lequel on hybride les primaires avec des portions de l'ADN matrice ayant un intervalle de 100 à 1000 bases.

6. Procédé selon la revendication 5, dans lequel l'intervalle entre les primaires est de 150 à 500 bases.

7. Procédé selon la revendication 4, dans lequel on hybride les primaires avec des portions de l'ADN matrice ayant des intervalles pratiquement uniformes.

8. Procédé selon la revendication 5, comprenant en outre le stade consistant à marquer un ADN à simple brin formé.

9. Procédé selon la revendication 1, dans lequel on effectue l'allongement du primaire en utilisant du triphosphate de désoxyribonucléoside ayant un marqueur ou un groupe fonctionnel auquel un marqueur peut être fixé.

10. Procédé selon la revendication 9, dans lequel l'allongement du primaire est effectué en utilisant un triphosphate de désoxyribonucléoside avec un groupe fonctionnel auquel un marqueur peut être fixé.

11. Procédé selon la revendication 10, dans lequel le groupe fonctionnel est un groupe amino ou un groupe carboxyle.

12. Procédé selon la revendication 10, dans lequel le marqueur est la biotine.

13. Procédé selon la revendication 1, dans lequel la séparation et la récupération de l'ADN à simple brin sont effectuées par électrophorèse sur un gel alcalin en utilisant une membrane de gel.

14. Procédé de préparation d'un ADN à simple brin comprenant les stades consistant à :
   - recombiner un vecteur de phage qui libère un phage sous forme d'un brin unique avec un ADN de matrice ayant une séquence de bases complémentaire de celle de l'ADN à brin unique à préparer;
   - transfecter une cellule hôte au moyen de l'ADN recombinant;
   - cultiver la cellule hôte pour libérer un ADN à simple brin qui est un recombinant du vecteur de phage et de l'ADN matrice;
   - hybrider un oligomère d'ADN avec la portion d'extrémité 5' de l'ADN matrice pour former une région à double brin à la portion d'extrémité 5' de l'ADN matrice;
   - couper la région à double brin ainsi formée avec une enzyme de restriction pour couper la portion d'extrémité 5' de l'ADN matrice;
   - hybrider plusieurs primaires avec des portions de l'ADN matrice qui sont espacées d'une manière pratiquement uniforme;

- allonger les primaires en utilisant l'ADN matrice comme matrice; et
- séparer et récupérer les ADN à simple brin formés par l'allongement des primaires.

restriction
enzyme

vector
(A)

(B)

recombination

DNA fragment
(C)

host
cell

(D)

(E)

DNA oligomer

restriction
enzyme

primer

(F)

(G)

(H)

extension

gel
membrane (J)

alkaline gel
electrophoresis

(I)

(K)